# EUROPÄISCHE PATENTSCHRIFT

(11) **EP 1 813 940 B1**
(45) Veröffentlichungstag und Bekanntmachung des Hinweises auf die Patenterteilung: **14.05.2014**
(21) Anmeldenummer: 07000392.6
(22) Anmeldetag: 10.01.2007
(51) Int. Cl.: G01N 33/20

(54) **Vorrichtung zum Bestimmen einer Kenngrösse einer Metallschmelze oder einer auf der Metallschmelze aufliegenden Schlackeschicht**
Device for determining characteristics of a metal melt or a slag layer on the metal melt
Dispositif pour déterminer la caractéristique d'un métal en fusion ou d'une couche de scories se trouvant sur le métal en fusion

(30) Priorität: 26.01.2006 DE 102006005476
(43) Veröffentlichungstag der Anmeldung: 01.08.2007
(73) Patentinhaber: Heraeus Electro-Nite International N.V., 3530 Houthalen (BE)
(72) Erfinder: Dams, Francis, 3010 Kessel-Lo (BE); Neyens, Guido Jacobus, 3680 Opoeteren (BE)
(74) Vertreter: Kühn, Hans-Christian

(56) Entgegenhaltungen:
- EP-A- 1 614 758
- WO-A-03/060432
- WO-A-2005/109012
- DE-A1- 2 354 298
- DE-A1- 3 347 479
- GB-A- 1 232 487
- US-A- 5 596 134
- US-A- 5 720 553
- ANONYMOUS: 'DS18S20 High-Precision 1-Wire Digital Thermometer' INTERNET CITATION, [Online] 21 Februar 2003, Seiten 1 - 21, XP55074239 Gefunden im Internet: <URL:https://wiki.har2009.org/w/images/1/13 /Ds18s20.pdf> [gefunden am 2013-08-06]

## Beschreibung

Die Erfindung betrifft eine Vorrichtung zum Bestimmen mindestens einer Kenngröße einer Metallschmelze (vorzugsweise Eisen- oder Stahlschmelze) oder einer auf der Metallschmelze aufliegenden Schlackeschicht mit einem Trägerrohr, an dessen einem Ende ein Messkopf angeordnet ist mit einem in dem Trägerrohr fixierten Körper, wobei im Inneren des Messkopfes oder des Trägerrohres ein A/D-Wandler angeordnet ist, wobei der A/D-Wandler mit mindestens einem an oder in dem Messkopf angeordneten Sensor verbunden ist.

Derartige Vorrichtungen sind aus WO 03/060432 A1 bekannt. Hier wird die Verwendung einer verbrauchbaren Messelektronik innerhalb einer Einwurfsonde beschrieben, wobei die Sensorsignale drahtlos an einer Auswertestation übertragen werden. Die Vorrichtung ist daher beim Fall in die Schmelze nicht durch Kabel behindert. Übliche Eintauchsonden, also Sonden, welche zur Messung oder Probennahme kurzzeitig in die Metallschmelze getaucht und dann wieder herausgezogen werden, sind mit ihrem Trägerrohr auf eine übliche, beispielsweise aus DE 29805881 U1, EP 69433, DE 3641225 A1 oder WO 03/064714 A1 bekannte Lanze (Messlanze) aufgesteckt, durch die hindurch die Ableitung der Messsignale erfolgt. In EP 1 614 758 A2 ist eine Messsonde für Metallschmelzen offenbart mit einem besonderen Lanzenhalter. Aus US 5,720,553 A ist ein Probennehmer mit einem Thermoelement bekannt, mit dem Eigenschaften einer Eisenschmelze bestimmt werden können mit einem dislozierten A/D-Wandler.

Der obengenanten Erfindung liegt die Aufgabe zugrunde, eine verbesserte Messvorrichtung zur Verfügung zu stellen, mit der eine genaue Messung von Kenngrößen einer Metallschmelze oder einer auf der Metallschmelze aufliegenden Schlackeschicht ermöglicht wird.

Die Aufgabe wird durch die Merkmale der unabhängigen Ansprüche gelöst. Vorteilhafte Ausgestaltungen sind in den Unteransprüchen angegeben.

Dadurch, dass der Messkopf ein Kontaktstück aufweist, welches mit einem Signalausgang und einem Stromversorgungsanschluss des A/D-Wandlers elektrisch leitend verbunden ist, dass das Kontaktstück mit einer in das Trägerrohr eingeführten Lanze verbunden ist und innerhalb der Lanze mindestens eine Signalleitung und mindestens eine Stromversorgungsleitung angeordnet sind, die einerseits mit dem Kontaktstück und andererseits mit einem Computer oder Auswertegerät verbunden sind, ist eine Weiterleitung der Messsignale von Sensoren als digitale Signale möglich, so dass elektrische, durch die Umgebung bedingte Störungen weitgehend eliminiert werden und eine Kompensation oder Abschirmung der Leitungen nicht notwendig ist. Analoge Signale werden nur über eine sehr kurze Stecke geführt, die Stromversorgung jedoch problemlos ermöglicht. Bei bekannten Lanzen/Messvorrichtungen vorhandene kritische Stellen, die Messengenauigkeiten oder Signalverfälschungen hervorrufen können, wie elektrische Kontakte und Verbindungen, werden durch die Digitale Technik und die Beschränkung der Zahl der Leitungen zumindest minimiert. Dabei können prinzipiell alle bekannten Sensoren eingesetzt werden, wie beispielsweise Temperatursensoren, elektrochemische Sensoren oder optische Sensoren.

Die Signale werden in über ein übliches Kontaktstück mit den durch eine Lanze geführten Leitungen verbunden, die an eine Auswerteeinheit angeschlossen sind. Die Lanze ist eine übliche Trägerlanze, auf die das Trägerrohr zur Messung aufgesteckt wird und mit der es während der Messung gehalten wird. Dabei ist es möglich, die Metallhülse der Lanze und eine in den Messkopf integrierte oder diesen umgebende Metallhülse, die mit der Metallhülse der Lanze verbunden ist, als eine von zwei Leitungen zu verwenden. Es braucht so nur eine Leitung durch die Lanze hindurch geführt werden. Die Messsignale und die Stromversorgung können wegen der Digitalisierung über eine einzige Leitung geführt werden.

Vorzugsweise ist das Kontaktstück mit einem Stromversorgungsanschluss des A/D-Wandiers elektrisch leitend verbunden. Erfindungsgemäß sind höchstens zwei Signalleitungen zur Weiterleitung digitaler Signale einerseits mit je einem Kontakt des Kontaktstückes und andererseits mit einem Computer oder Auswertegerät verbunden.

Erfindungswesentlich ist es, dass innerhalb der Lanze eine Stromversorgungsleitung angeordnet sein kann, die einerseits mit einem Kontakt des Kontaktstückes und andererseits mit einer Stromquelle verbunden ist.

Innerhalb der Lanze ist eine einerseits mit einem Kontakt des Kontaktstückes und andererseits mit einem Mess- oder Auswertegerät verbundene Signalleitung angeordnet und eine zweite Signalleitung durch das Metallrohr der Lanze gebildet, die mit einem Kontakt des Kontaktstückes elektrisch verbunden ist. Die innerhalb der Lanze angeordnete Signalleitung ist auch als Stromversorgungsleitung ausgebildet und mit einer Stromquelle verbunden.

Der A/D-Wandler kann auf einer Leiterplatte (oder einem Leiterrahmen oder einer anderen Einrichtung zur Aufnahme elektrischer Bauelemente) angeordnet sein.

In der erfindungsgemäßen Vorrichtung ist prinzipiell die gesamte Messelektronik als Einwegmaterial ausgelegt, welches nach einmaligem Gebrauch zusammen mit dem Messkopf und dem Trägerrohr entsorgt wird. Der Messkopf kann weitere Sensoren tragen, beispielsweise Sauerstoffsensoren, optische Sensoren oder Temperatursensoren, die in üblicherweise über das Kontaktstück mit einer Auswerteelektronik verbunden werden können.

Nachfolgend wird ein Ausführungsbeispiel der Erfindung an Hand einer Zeichnung näher erläutert. In der Zeichnung zeigt:
- Fig. 1: eine Sublanze
- Fig. 2: eine weitere Sublanze
- Fig. 3: eine erfindungsgemäße Ausführungsform einer Sublanze
- Fig. 4: eine erfindungsgemäße Wurfsonde
- Fig. 5: eine zweite Ausführungsform einer Wurfsonde und
- Fig. 6: die Wurfsonde nach Fig. 5, während der Messung in der Schmelze.

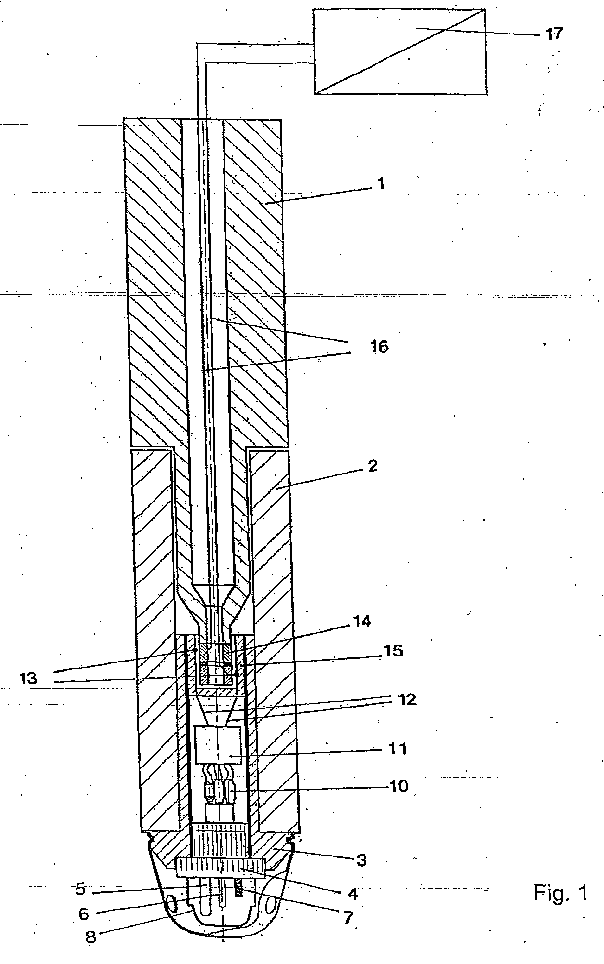

Beispiele in Fig. 1, 2, 4, 5 und 6 stellen lediglich Illustrationen des technischen Hintergrunds das.

Die in Fig. 1 dargestellte Vorrichtung betrifft eine sogenannte Sublanze. An einer Lanze 1 ist ein Trägerrohr 2 mit einem Messkopf 3 angeordnet. Das Trägerrohr 2 ist aus Pappe gebildet, der Messkopf 3 im wesentlichen aus einem feuerfesten Material wie Zement oder Gießereisand. Der Messkopf 3 weist einen Sensorträger 4 auf, an dessen äußerem Ende, dem Eintauchende ein Sauerstoffsensor 5, ein Temperatursensor 6 und der sogenannte Badkontakt 7 angeordnet sind. Die Sensoren sind mit einer ersten Schutzkappe 8, welche an dem Sensorträger 4 fixiert ist, bis zum Beginn der Messung geschützt. Der gesamte aus dem Trägerrohr 2 herausragende Teil des Messkopfes 3 ist von einer weiteren Schutzkappe 9 umgeben. Am rückseitigen, im inneren des Messkopfes 3 angeordneten Ende des Sensorträgers 4 ist eine sogenannte Drahtverbindung 10 angeordnet, die die Sensoren 5; 6; 7 mit einem A/D-Wandler 11 verbinden. Der A/D-Wandler 11 weist an seinem anderen, ausgangsseitigen Ende zwei Signalleitungen 12 auf, die mit entsprechenden Kontakten 13 eines Kontaktstückes 14 verbunden sind. Das Kontaktstück 14 ist an einen mechanischen Konnektor 15 (der der mechanischen Verbindung der Lanze 1 mit dem Messkopf 3 dient) angeordnet. Am rückseitigen Ende des Kontaktstückes 14 sind zwei Signalleitungen 16 angeordnet, die durch die Lanze 1 hindurch mit einer Stromquelle bzw. einem Auswertegerät 17 verbunden sind.

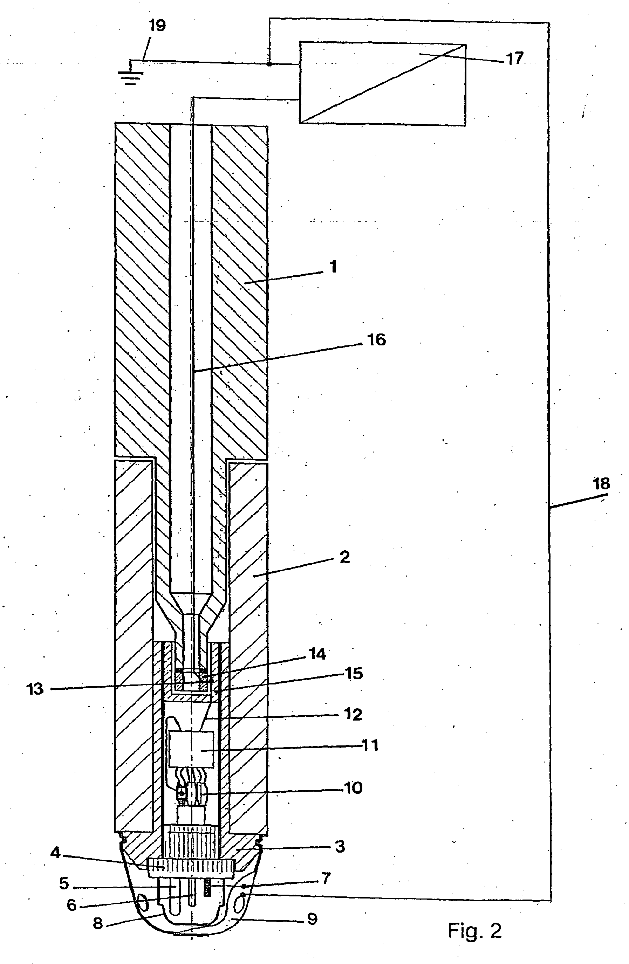

Die in Fig. 2 dargestellte Sublanze ist ähnlich der in Fig. 1 dargestellten Sublanze aufgebaut. Im Unterschied dazu wird der A/D-Wandler 11 durch nur eine Signalleitung 12 mit dem Kontaktstück 14 verbunden und vom Kontaktstück 14 wird nur eine Signalleitung 16 durch das Trägerrohr 1 hindurch zu einem Auswertegerät 17 bzw. einer Stromquelle geführt. Die zweite zur Bildung eines Stromkreises notwendige Leitung wird vom Badkontakt gebildet, der nach dem Eintauchen in die Metallschmelze über diese Metallschmelze mittels der in Fig. 2 symbolisch dargestellten Signalleitung 18 (entspricht der Metallschmelze) auf Massepotential 19 gelegt ist. Das Massepotential 19 ist mit dem Auswertegerät 17 verbunden.

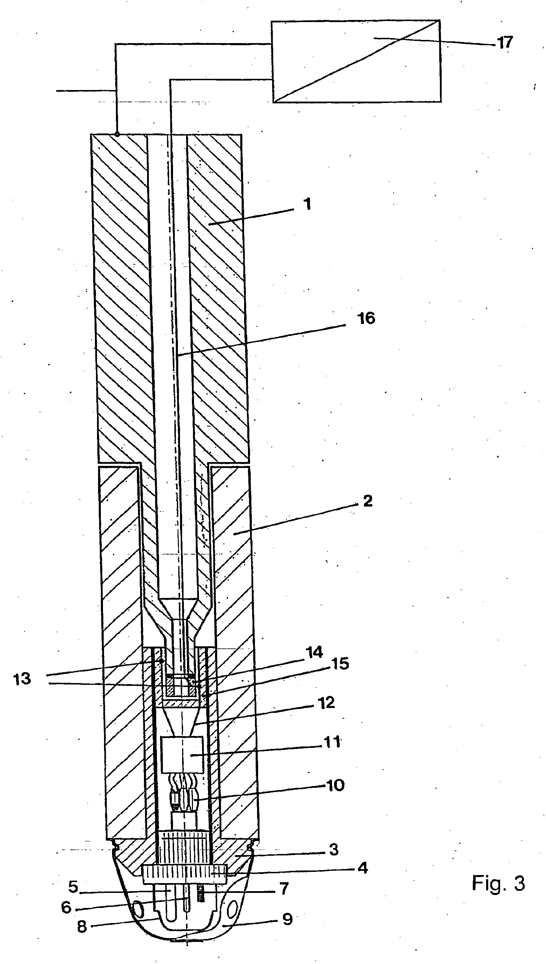

Die in Fig. 3 dargestellte erfindungsgemäße Sublanze ist ebenfalls ähnlich zur Fig. 1 aufgebaut. Im Unterschied zu Fig. 1 ist das Kontaktstück 14 an seinem dem A/D-Wandler 11 angewandten Ende mit einer durch die Lanze 1 geführten Signalleitung 16 sowie mit der Lanze 1 selbst elektrisch verbunden. Die Lanze 1 ist zumindest zum Teil aus Metall gebildet, also leitfähig und an ihrem dem Eintauchende abgewandten Ende durch eine Masseleitung 20 mit dem Auswertegerät 17 verbunden.

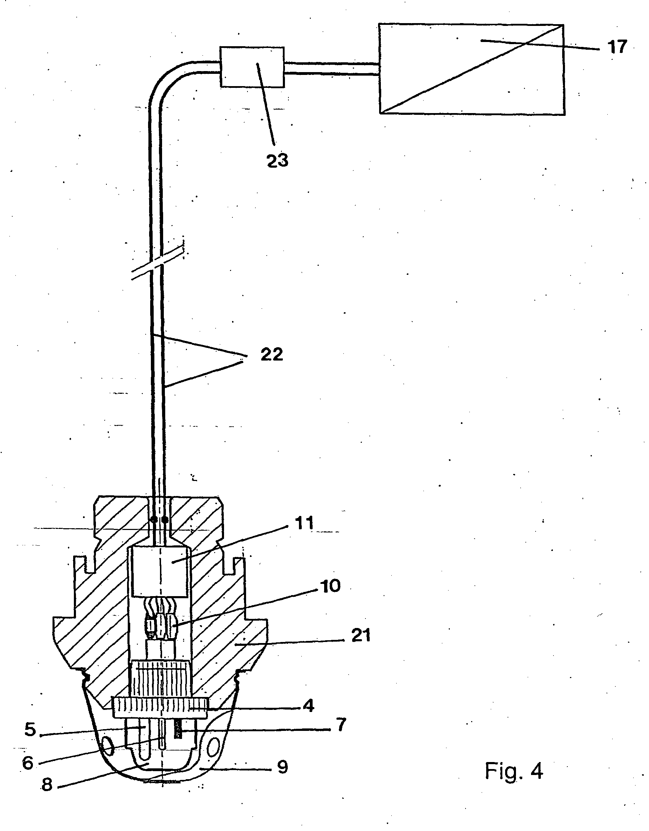

In Fig. 4 ist ein sogenannter Wurfsensor 21 dargestellt. Der Wurfsensor 21 wird aus größerer Höhe, meist automatisch, in die Metallschmelze abgeworfen. Der Wurfsensor 21 ist über Signalkabel 22 mit einem Kontaktstück 23 verbunden, welches in der Regel in der Nähe der Abwurfstelle angeordnet und mit dem Auswertegerät 17 bzw. der Stromquelle verbunden ist. Der innere Aufbau des Wurfsensors 21 mit den Sensoren entspricht im wesentlichen dem Aufbau des Messkopfes nach Fig. 1.

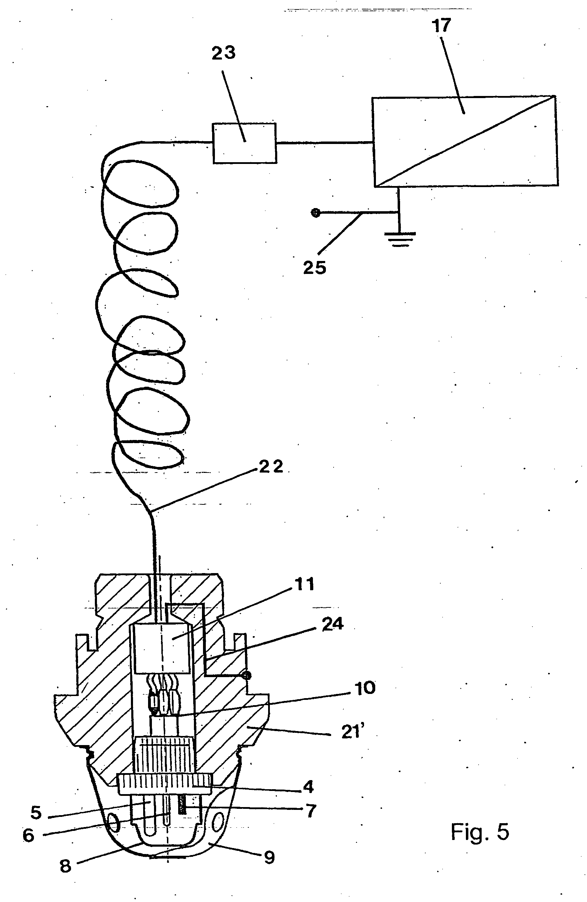

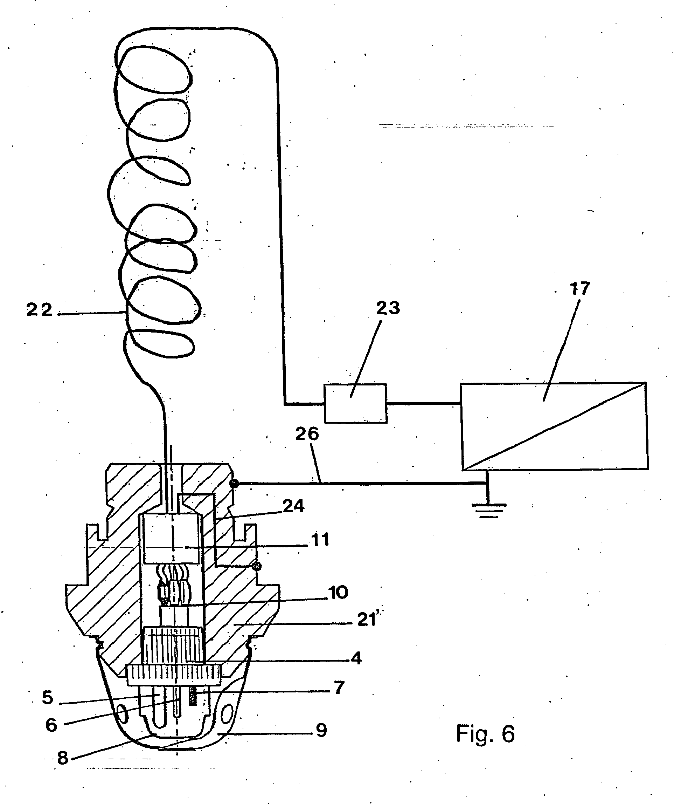

Der in Fig. 5 dargestellte Wurfsensor 21' ist ähnlich aufgebaut. Er weist allerdings nur eine, vom A/D-Wandler wegführende Signalleitung 22 auf. Die zweite Leitung, die Masseleitung, wird ähnlich der Anordnung in Fig. 2 nach dem Eintauchen des Wurfsensors 21' in die Metallschmelze über diese realisiert (Fig. 6). Dazu ist innerhalb des Wurfsensors 21' der A/D-Wandler an seinem dem Sensorträger abgewandten Ende (abgewandt im Sinne der elektrischen Verbindung) neben der Signalleitung 22 mit einem Massekontakt 24 verbunden, der durch das Material des Wurfsensors 21' hindurch mit der Metallschmelze verbunden ist. Die Metallschmelze liegt auf Massepotential, so dass sie über die Masseleitung 25 des Auswertegerätes 17 mit diesem verbunden ist. Diese Art der Kontaktierung ist also ähnlich dem in Fig. 2 dargestellten Kreislauf aufgebaut. In Fig. 6 ist der Wurfsensor 21' während der Messung in der Stahlschmelze (diese ist in der Zeichnung nicht gezeigt) dargestellt. Die Masseleitung durch die Stahlschmelze ist mit der Bezugszahl 26 bezeichnet. Sie verbindet den Wurfsensor 21' mit dem Auswertegerät 17.

Der Körper des Wurfsensors 21; 21' besteht aus Stahl, um die zum Durchdringen der auf der Metallschmelze (z.B. eine Stahlschmelze) aufliegende Schlacke zu gewährleisten bzw. um eine Kontaktierung gemäß Fig. 5 und 6 zu ermöglichen.

## Patentansprüche

1. Vorrichtung zum Bestimmen mindestens einer Kenngröße einer Eisen- oder Stahlschmelze oder einer auf der Eisen- oder Stahlschmelze aufliegenden Schlackeschicht, aufweisend ein Trägerrohr (2), eine Lanze (1), eine Stromquelle und einen Computer oder ein Auswertegerät (17), wobei an einem Ende des Trägerrohres (2) ein Messkopf (3) angeordnet ist mit einem in dem Trägerrohr fixierten Körper, wobei im Inneren des Messkopfes oder des Trägerrohres ein A/D-Wandler angeordnet ist, wobei der A/D-Wandler (11) mit mindestens einem an oder in dem Messkopf angeordneten Sensor (5,6,7) verbunden ist, **dadurch gekennzeichnet, dass** der Messkopf (3) ein Kontaktstück (14) aufweist, welches über seine Kontakte (13) mit einem Signalausgang des A/D-Wandlers (11) elektrisch leitend verbunden ist, dass das Kontaktstück (14) mit einer in das Trägerrohr (2) eingeführten Lanze (1) verbunden ist und dass höchstens zwei Signalleitungen (16) zur Weiterleitung digitaler Signale einerseits über je einen Kontakt des Kontaktstückes (14) mit dem A/D-Wandler(11) und andererseits mit einem Computer oder einem Auswertegerät (17) verbunden sind, wobei innerhalb der Lanze (1) eine einerseits mit einem Kontakt des Kontaktstückes (14) und andererseits mit einem Computer oder einem Auswertegerät (17) verbundene Signalleitung (16) angeordnet ist und zusätzlich auch als Stromversorgungsleitung ausgebildet ist und mit einer Stromquelle verbunden ist, und die zweite Signalleitung durch das Metallrohr der Lanze (1) gebildet ist, die mit einem Kontakt des Kontaktstückes (14) elektrisch verbunden ist.

2. Vorrichtung nach Anspruch 1, **dadurch gekennzeichnet, dass** das Kontaktstück (14) mit einem Stromversorgungsanschluß des A/D-Wandlers (11) elektrisch leitend verbunden ist.

3. Vorrichtung nach einem der Ansprüche 1 bis 2, **dadurch gekennzeichnet, dass** der A/D-Wandler (11) auf einer Leiterplatte angeordnet ist.

## Claims

1. Apparatus for determining at least one characteristic of molten iron or molten steel or of a slag layer resting on the molten iron or molten steel comprising a support tube (2), a lance (1), an electrical power source and a computer or an analytical unit (17), whereby a measuring head (3) is arranged on one end of the support tube (2) and has a body that is fixed inside the support tube, whereby an A / D converter (11) is arranged inside the measuring head or support tube, whereby the A / D converter (11) is connected to at least one sensor (5, 6, 7) that is arranged on or in the measuring head, **characterised in that** the measuring head (3) comprises a contact piece (14) which is connected in electrically conductive manner via its contacts (13) to a signal output of the A / D converter (11), **in that** the contact piece (14) is connected to a lance (1) that is inserted into the support tube (2) and **in that** at most two signal lines (16) for transmitting digital signals further are connected, on the one hand, via one contact each of the contact piece (14) to the A / D converter (11) and, on the other hand, to a computer or an analytical unit (17), whereby a signal line (16), which is connected, on the one hand, to a contact of the contact piece (14) and, on the other hand, to a computer or an analytical unit (17), is arranged inside the lance (1) and is, in addition, provided as an electrical power supply line and is connected to an electrical power source, and the second signal line is formed by the metal tube of the lance (1) that is connected in electrically conductive manner to a contact of the contact piece (14).

2. Apparatus according to claim 1, **characterised in that** the contact piece (14) is connected in electrically conductive manner to an electrical power supply terminal of the A / D converter (11).

3. Apparatus according to any one of the claims 1 to 2, **characterised in that** the A / D converter (11) is arranged on a printed circuit board.

## Revendications

1. Dispositif de détermination d'au moins un paramètre d'une fonte de fer ou d'acier ou d'une couche de scorie couchée sur la fonte de fer ou d'acier, présentant un tuyau de support (2), une lance (1), une source d'électricité et un ordinateur ou un appareil d'évaluation (17), dans lequel une tête de mesure (3) est disposée à une extrémité du tuyau de support (2) avec un corps fixé dans le tuyau de support, dans lequel un convertisseur analogique-numérique (11) est disposé à l'intérieur de la tête de mesure ou du tuyau de support, dans lequel le convertisseur analogique-numérique (11) est relié à au moins un capteur (5, 6, 7) disposé sur ou dans la tête de mesure, **caractérisé en ce que** la tête de mesure (3) présente une pièce de contact (14) qui est reliée de manière électriquement conductrice par le biais de ses contacts (13) à une sortie de signal du convertisseur analogique-numérique (11), que la pièce de contact (14) est reliée à une lance (1) introduite dans le tuyau de support (2) et que deux fils de signaux (16) maximum sont reliés d'un côté par le biais d'un contact de la pièce de contact (14) à chaque fois au convertisseur analogique-numérique (11) et de l'autre côté à un ordinateur ou un appareil d'évaluation (17) pour la transmission de signaux numériques, dans lequel un fil de signaux (16) relié d'un côté à un contact de la pièce de contact (14) et de l'autre côté à un ordinateur ou un appareil d'évaluation (17) est disposé à l'intérieur de la lance (1), et est en outre également réalisé en tant que conduite d'alimentation électrique et est relié à une source d'électricité, et le second fil de signaux est formé par le tuyau métallique de la lance (1) qui est reliée électriquement à un contact de la pièce de contact (14).

2. Dispositif selon la revendication 1, **caractérisé en ce que** la pièce de contact (14) est reliée de manière électriquement conductrice à un raccord d'alimentation électrique du convertisseur analogique-numérique (11).

3. Dispositif selon l'une quelconque des revendications 1 à 2, **caractérisé en ce que** le convertisseur analogique-numérique (11) est disposé sur une carte de circuits imprimés.
